(19) Europäisches Patentamt
European Patent Office
Office européen des brevets

(11) Veröffentlichungsnummer: **0 001 105**
**A1**

(12) # EUROPÄISCHE PATENTANMELDUNG

(21) Anmeldenummer: **78100817.2**

(22) Anmeldetag: **04.09.78**

(51) Int. Cl.²: **C 07 D 295/12**

(30) Priorität: **07.09.77 DE 2740208**

(43) Veröffentlichungstag der Anmeldung:
**21.03.79 Patentblatt 79/6**

(84) Benannte Vertragsstaaten:
**BE DE FR GB**

(71) Anmelder: **BASF Aktiengesellschaft**
**Carl-Bosch-Strasse 38**
**D-6700 Ludwigshafen(DE)**

(72) Erfinder: **Weitz, Hans-Martin, Dr.**
**Auf dem Koeppel 40**
**D-6702 Bad Duerkheim 1(DE)**

(72) Erfinder: **Fischer, Rolf, Dr.**
**Bergstrasse 98**
**D-6900 Heidelberg(DE)**

(54) **Verfahren zur Herstellung von 2-Piperidino-3-hydroxyl-amino-cyclohexanonoximen.**

(57) 2-Piperidino-3- hydroxylamino-cyclohexanonoxime, Verfahren zur Herstellung von 2-Piperidino-3- hydroxylamino - cyclohexanonoximen der Formel

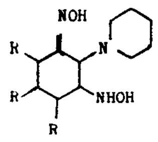

(I)

worin die einzelnen Rest R gleich oder verschieden sein können und jeweils ein Wasserstoffatom, einen aliphatischen, cycloaliphatischen, araliphatischen oder aromatischen Rest bedeuten darüber hinaus zwei benachbarte Reste R auch zusammen mit den beiden benachbarten Kohlenstoffatomen Glieder eines Ringes bezeichnen können, durch Umsetzung von den entsprechenden 2-Piperidino-cyclohexen -(2)-onen mit Hydroxylamin.

Die Verbindungen I stellen Komplexbildner für Schwermetallionen dar, sie sind Ausgangsstoffe für die Herstellung von Triaminen, die als alkalische Katalysatoren, Zusätze zu Epoxidharzen und als Ausgangsstoffe für die Synthese von Farbstoffen und Arzneimittel dienen.

EP 0 001 105 A1

BASF Aktiengesellschaft O. Z. 0050/032780

BEZEICHNUNG GEÄNDERT
siehe Titelseite

Verfahren zur Herstellung von 2-Piperidino-3-hydroxylaminocyclohexanonoximen

Die Erfindung betrifft ein Verfahren zur Herstellung von 2-Piperidino-3-hydroxylamino-cyclohexanonoximen durch Umsetzung von 2-Piperidino-cyclohexen-(2)-onen mit Hydroxylamin.

Es ist bekannt, daß man 1,2-Dioximinocyclohexan durch Umsetzung von Cyclohexan-1,2-dion (Organic Synthesis, Collective Volume IV, Seiten 229 bis 232 (1967)) oder 2-Oximinocyclohexanon (Journal of Organic Chemistry, Band 11, Seiten 771 bis 772 (1946)) mit Hydroxylamin erhält.

Es wurde nun gefunden, daß man 2-Piperidino-3-hydroxylamino-cyclohexanonoxime der Formel

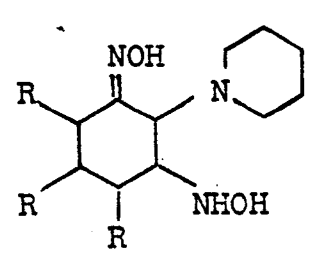

I,

WB/Fe

worin die einzelnen Reste R gleich oder verschieden sein können und jeweils ein Wasserstoffatom, einen aliphatischen, cycloaliphatischen, araliphatischen oder aromatischen Rest bedeuten, darüber hinaus zwei benachbarte Reste R auch zusammen mit den beiden benachbarten Kohlenstoffatomen Glieder eines Ringes bezeichnen können, vorteilhaft erhält, wenn man 2-Piperidino-cyclohexen-(2)-one der Formel

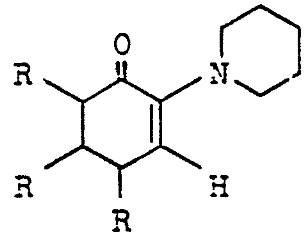

II,

worin R die vorgenannte Bedeutung besitzt, mit Hydroxylamin umsetzt.

Weiterhin wurden die neuen 2-Piperidino-3-hydroxylamino-cyclohexanonoxime der Formel

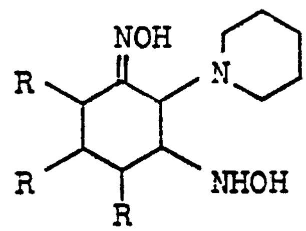

I,

worin die einzelnen Rest R gleich oder verschieden sein können und jeweils ein Wasserstoffatom, einen aliphatischen, cycloaliphatischen, araliphatischen oder aromatischen Rest bedeuten, darüber hinaus zwei benachbarte Reste R auch zusammen mit den beiden benachbarten Kohlenstoffatomen Glieder eines Ringes bezeichnen können, gefunden.

Die Umsetzung kann für den Fall der Verwendung von 2-Piperidino-cyclohexen-(2)-on durch die folgenden Formeln wiedergegeben werden:

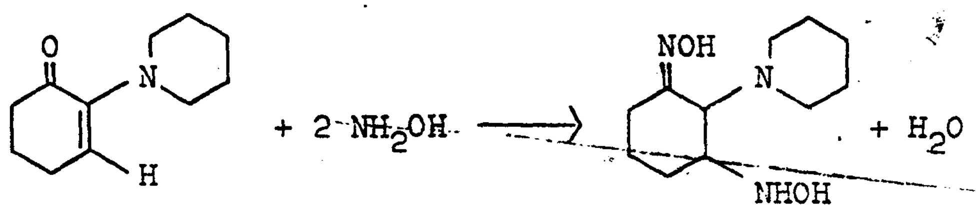

Das Verfahren nach der Erfindung liefert auf einfachem und wirtschaftlichem Wege die neuen 2-Piperidino-3-hydroxylamino-cyclohexanonoxime in guter Ausbeute, Reinheit sowie Raum-Zeit-Ausbeute.

Die Ausgangsstoffe II können durch Umsetzung der entsprechenden 1,2-Diketone (Zhurnal Organicheskoi Khimii, Band 9, Seiten 2 254 bis 2 256 (1973)) oder von 2,3-Epoxycycloalkanonen (Journal of Organic Chemistry, Band 35, Seiten 1 709 bis 1 711 (1970)) mit Piperidin erhalten werden. Sie werden in stöchiometrischer Menge oder im Überschuß mit Hydroxylamin, zweckmäßig in einer Menge von 2 bis 3 Mol, vorzugsweise 2,1 bis 2,5 Mol Hydroxylamin je Mol Ausgangsstoff II, umgesetzt. Bevorzugte Ausgangsstoffe II und dementsprechend bevorzugte Endstoffe I sind solche, in deren Formeln die einzelnen Reste R gleich oder verschieden sein können und jweils ein Wasserstoffatom, einen Alkylrest mit 1 bis 6 Kohlenstoffatomen, einen Cyclohexylrest, einen Aralkylrest mit 7 bis 12 Kohlenstoffatomen, einen Phenylrest bedeuten, darüber hinaus auch zwei benachbarte Reste R zusammen mit den beiden benachbarten Kohlenstoffatomen Glieder eines 5- oder 6-gliedrigen, alicyclischen Ringes bezeichnen können. Die vorgenannten Reste und Ringe können noch durch unter den Reaktionsbedingungen inerte Gruppen, z.B. Alkylgruppen oder Alkoxygruppen mit jeweils 1 bis 4 Kohlenstoffatomen, substituiert sein.

So sind beispielsweise folgende Stoffe als Ausgangsstoffe II geeignet: In 4-Stellung, 5-Stellung oder 6-Stellung einfach oder in 4,5- bzw. in 5,6- bzw. in 4,6-Stellung zweifach oder in 4,5,6-Stellung dreifach durch Methyl-, Äthyl-, n-Propyl-, Isopropyl-, n-Butyl-, Isobutyl-, sek.-Butyl-, tert.-Butyl-, Cyclohexyl-, Benzyl-, Phenyl-gruppen substituierte 2-Piperidino-cyclohexen-(2)-one; bevorzugt unsubstituiertes 2-Piperidino-cyclohexen-(2)-on.

Die Umsetzung wird im allgemeinen bei einer Temperatur von -20 bis +150°C, vorzugsweise von +10 bis +90°C, drucklos oder unter Druck, diskontinuierlich oder kontinuierlich durchgeführt. Im allgemeinen verwendet man zusätzlich unter den Reaktionsbedingungen inerte Lösungsmittel, zweckmäßig solche, in denen Hydroxylamin löslich ist. Als Lösungsmittel kommen z.B. in Frage: Alkanole und Cycloalkanole wie Äthanol, Methanol, n-Butanol, Isobutanol, tert.-Butanol, Glykol, Glycerin, n-Propanol, Isopropanol, Amylalkohol, Cyclohexanol, 2-Methyl4-pentanol, Äthylenglykolmonoäthyläther, 2-Äthylhexanol, Methylglykol, n-Hexanol, Isohexylalkohol, Isoheptylalkohol, n-Heptanol, Äthylbutanol, Nonylalkohol, Dodecylalkohol, Methylcyclohexanol, Diacetonalkohol, insbesondere solche mit 1 bis 6 Kohlenstoffatomen; und entsprechende Gemisch. Zweckmäßig verwendet man das Lösungsmittel in einer Menge von 200 bis 10 000 Gewichtsprozent, vorzugsweise von 400 bis 2 000 Gewichtsprozent, bezogen auf Ausgangsstoff II.

Das Hydroxylamin wird in der Regel in Form seiner Salze verwendet. Geeignete Salze sind z.B. das Chlorid, Nitrat, Sulfat, Formiat oder Acetat des Hydroxylamins. Ebenfalls ist es möglich, anstelle der Salze Hydroxylamin selbst dem Reaktionsgemisch zuzuführen. Zweckmäßig stelle man, z.B. in der in Kirk-Othmer, Encyclopedia of Chemical Technology, Second Edition, Band 11, Seite 497 beschriebenen Arbeitsweise, eine Lösung von freiem Hydroxylamin in Wasser und/oder Alkohol her; als Alkohol verwendet man im allgemeinen die vorgenannten, insbesondere Methanol, Äthanol, n-Propanol oder n-Butanol. Man kann auch aus einem Hydroxylammoniumsalz, beispielsweise Hydroxylammoniumchlorid, Hydroxylammoniumsulfat, Hydroxylammoniumnitrat oder Hydroxylammoniumacetat, das in einem der genannten Lösungsmittel gelöst ist, mit Basen wie Natriumhydroxid, Kaliumhydroxid, Calciumhydroxid, Ammoniak, Natriumcarbonat, Natriummethylat oder Natriumäthylat Hydroxylamin freisetzen. Das anfallende Natrium-, Kalium-, Ammonium- oder Calciumsalz wird vor oder nach der Umsetzung mit Ausgangsstoff II abfiltriert.

Die Reaktion kann wie folgt durchgeführt werden: Ein Gemisch von Ausgangsstoff II, Hydroxylamin und in der Regel Lösungsmittel wird bei der Reaktionstemperatur 1 bis 20 Stunden gehalten. Die Hydroxylaminlösung wird in der Regel unter Rühren zu einer Lösung des Ausgangsstoffes II in einem der genannten Lösungsmittel hinzugegeben. Es ist auch möglich, ein Gemisch aus Hydroxylammoniumsalz, Base und Ausgangsstoff II in einem der genannten Lösungsmittel ohne Herstellung einer Hydroxylaminlösung direkt umzusetzen. Der Endstoff I wird nach der Umsetzung in üblicher Weise, z.B. durch Filtration, gegebenenfalls nach Fällung, beispielsweise mit Wasser, und gegebenenfalls Umkristallisation, abgetrennt.

Die nach dem Verfahren der Erfindung herstellbaren Verbindungen I stellen Komplexbildner für Schwermetallionen dar. Sie sind wertvolle Ausgangsstoffe für die Herstellung von Triaminen, z.B. durch Reduktion der Oximino- und Hydroxylaminogruppe mit katalytisch aktiviertem Wasserstoff, Lithiumaluminiumhydrid oder Zink (Houben-Weyl, Methoden der Organischen Chemie, Band 11, Teil 1, Seiten 495 bis 502). Derartige Triamine dienen als alkalische Katalysatoren, Zusätze zu Epoxidharzen und alsAusgangsstoffe für die Synthese von Farbstoffen und Arzneimitteln.

Die in dem folgenden Beispiel aufgeführten Teile bedeuten Gewichtsteile.

Beispiel

47,5 Teile Kaliumhydroxid, gelöst in 105 Teilen Methanol, werden mit einer warmen Lösung (50°C) von 58,8 Teilen Hydroxylammoniumchlorid in 213 Teilen Methanol versetzt. Das beim Abkühlen ausfallende Kaliumchlorid wird abfiltriert. Zum Filtrat werden innerhalb von 15 Minuten 60 7 Teile 2-Piperidino-cyclohexen-(2)-on bei 20°C zugegeben. Das Reaktionsgemisch wird 14 Stunden bei 23°C gerührt. Nach Filtration des Gemisches und Trocknen des Festgutes werden 53,2 Teile 2-Piperidino-3-hydroxylamino-cyclohexanonoxim (69,1 % der Theorie) erhalten. Schmelzpunkt 168 bis 169°C (Zers.) (aus Äthanol).

## Patentansprüche

1. Verfahren zur Herstellung von 2-Piperidino-3-hydroxylamino-cyclohexanonoxim der Formel

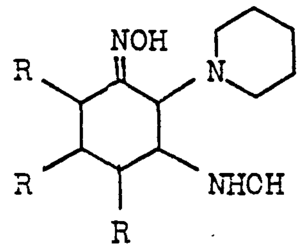

I,

worin die einzelnen Reste R gleich oder verschieden sein können und jeweils ein Wasserstoffatom, einen aliphatischen, cycloaliphatischen, araliphatischen oder aromatischen Rest bedeuten, darüber hinaus benachbarte Reste R auch zusammen mit den beiden benachbarten Kohlenstoffatomen Glieder eines Ringes bezeichnen können, <u>dadurch gekennzeichnet</u>, daß man 2-Piperidino-cyclohexen-(2)-one der Formel

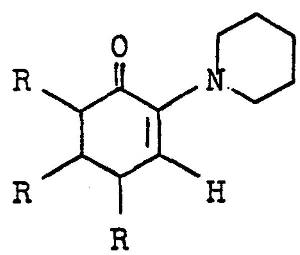

II,

worin R die vorgenannte Bedeutung besitzt, mit Hydroxylamin umsetzt.

2. 2-Piperidino-3-hydroxylamino-cyclohexanonoxime der Formel

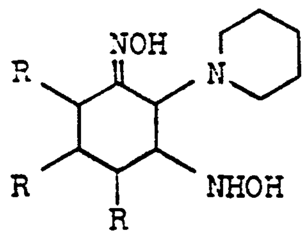

I,

worin die einzelnen Reste R gleich oder verschieden sein können und jeweils ein Wasserstoffatom, einen aliphatischen, cycloaliphatischen, araliphatischen oder aromatischen Rest bedeuten, darüber hinaus zwei benachbarte Reste R auch zusammen mit den beiden benachbarten Kohlenstoffatomen Glieder eines Ringes bezeichnen können.

Europäisches Patentamt

# EUROPÄISCHER RECHERCHENBERICHT

0001105

Nummer der Anmeldung
EP 78 10 0817

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| | BERICHTE DER DEUTSCHEN CHEMISCHEN GESELLSCHAFT, Jahrgang 32 (1899) Seiten 1340-1342<br>* Seiten 1340, 1342 *<br>---- | 1 | C 07 D 295/12 |
| | | | RECHERCHIERTE SACHGEBIETE (Int. Cl.²)<br>C 07 D 295/12 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| ✓ | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Den Haag | 01-12-1978 | PAUWELS |